Europäisches Patentamt

European Patent Office

Office européen des brevets

(11) Veröffentlichungsnummer: **0 228 046 B1**

(12) **EUROPÄISCHE PATENTSCHRIFT**

(19)

(45) Veröffentlichungstag der Patentschrift: **25.03.92**

(21) Anmeldenummer: **86117749.1**

(22) Anmeldetag: **19.12.86**

(51) Int. Cl.⁵: **G01N 33/533**, //C12Q1/28, G01N21/76

(54) Verfahren zur Steigerung der Ouanten-Ausbeute bei der Oxidation von Luminol durch Peroxide in Gegenwart von Peroxidase.

(30) Priorität: **20.12.85 DE 3545398**

(43) Veröffentlichungstag der Anmeldung:
**08.07.87 Patentblatt 87/28**

(45) Bekanntmachung des Hinweises auf die
Patenterteilung:
**25.03.92 Patentblatt 92/13**

(84) Benannte Vertragsstaaten:
**AT BE CH DE ES FR GB GR IT LI LU NL SE**

(56) Entgegenhaltungen:
**EP-A- 0 087 959**
**EP-A- 0 116 454**
**EP-A- 0 157 629**
**GB-A- 2 162 946**

**PATENT ABSTRACTS OF JAPAN, Band 7, Nr.
282 (P-243)[1427], 16. Dezember 1983**

**NATURE, Band 305, Nr. 5930, September
1983, Chesham, Bucks (GB); T.P.WHITEHEAD
et al., Seiten, 158-159**

(73) Patentinhaber: **BOEHRINGER MANNHEIM
GMBH
Patentabteilung, Abt. E Sandhofer Strasse
112-132 Postfach 31 01 20
W-6800 Mannheim 31 Waldhof(DE)**

(72) Erfinder: **Wulff, Karl, Dr.
792 Winter Court
Carmel, IN 46032(US)**
Erfinder: **Gerber, Marin, Dr.
Ignaz Manz-Str. 1
W-8120 Weilheim-Unterhausen(DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Kopernikusstrasse 9 Postfach 86 08
20
W-8000 München 86(DE)**

CHEMICAL ABSTRACTS, Band 87, Nr. 1, 04 Juli 1977, Columbus, OH (US); J.NIKOKAVOURAS et al., S. 396, Nr. 4862b

CHEMICAL ABSTRACTS, Band 105, Nr. 8, 25. August 1986, Columbus, OH (US); J.LASOVSKY et al., S. 582, Nr. 69401k

CHEMICAL ABSTRACTS, Band 104, Nr. 18, 05 Mai 1986, Columbus, OH (US); C.D.KALKAR et al., S. 631, Nr. 158624g

**Beschreibung**

Die Erfindung betrifft ein Verfahren zur Steigerung der Quantenausbeute der Chemilumineszenz bei der Oxidation von Luminol oder Luminolderivaten durch Peroxidverbindungen in Gegenwart von Peroxidase (POD).

Chemilumineszenzreaktionen sind Vorgänge, bei denen ein fluoreszenzfähiges Molekül durch chemische Energie in einen angeregten Elektronenzustand gebracht wird, aus dem dann Energie als sichtbares Licht abgegeben wird. Biolumineszenzreaktionen sind enzymatisch katalysierte Chemilumineszenzreaktionen, bei denen Sauerstoff als Elektronenakzeptor wirkt. Die Quantenausbeuten (Verhältnis von Chemilumineszenz-Lichtquanten pro umgesetzte Moleküle) liegen allerdings nur bei etwa 1 % (vgl. K.D. Gundermann, Angew. Chemie 77 (1965) 572 bis 580; Chemiker-Zeitung 99 (1975) Nr. 6, 279 bis 285).

Die durch Peroxidase (POD) katalysierte Reaktion von Luminol (3-Amino-phthalsäurehydrazid) oder von Luminolderivaten mit Peroxiden wird als Indikatorreaktion in Immuno-Assays verwendet, wobei entweder POD oder Luminol als Marker dienen kann. Peroxidase (POD; Donor: $H_2O_2$-Oxidoreduktase, EC 1.11.1.7) bezeichnet eine Gruppe von Enzymen, welche die Oxidation einer großen Anzahl organischer Verbindungen katalysieren.

Bedeutung hat die POD-Bestimmung vor allem in Verbindung mit vorgeschalteten Reaktionen, bei denen $H_2O_2$ gebildet wird, beispielsweise für die Blutzuckerbestimmung sowie bei enzym-immunologischen Bestimmungsverfahren, die POD als Markierungsenzym verwenden, erlangt. Andere Analysenmethoden, bei denen die POD-Bestimmung von Bedeutung ist, sind beispielsweise die Galactose-, Wasserstoff-peroxid-, Katalase- oder Oxidasenbestimmung.

Es ist bekannt, POD durch Abnahme des $H_2O_2$ oder des Wasserstoffdonators sowie durch Entstehung der oxidierten Verbindung zu messen. Besondere Bedeutung hat dabei die letztere Methode gefunden, wobei als Substrate beispielsweise Di-o-anisidin, Guajacol oder ABTS (2,2′-Azinodi [3-äthyl-benzthiazolin-(6)-sulfonsäure]) verwendet werden.

Diese bekannten Methoden haben sich zwar bewährt, es besteht jedoch ein Bedarf an Methoden höherer Empfindlichkeit, um die POD-Bestimmung im Rahmen von Enzym-Immun-Tests zu verkürzen. POD spielt z.B. als Markierungsenzym bei den sog. "Enzym-Immuno-Assays" nach dem ELISA-Prinzip (enzyme-linked immuno sorbent assay) eine große Rolle. Mit den zahlreichen handelsüblichen Testreagenzien, die auf diesem System beruhen, ist die Dauer der eigentlichen POD-Bestimmung, z.B. mit ABTS als Substrat, die bei ca. 60 Minuten liegt, aber äußerst unbefriedigend.

Zur Lösung dieses Problems wurde versucht, die durch Peroxidase katalysierte Reaktion von Luminol mit Peroxidverbindungen durch Erhöhung der Quantenausbeute empfindlicher zu machen.

Aus der DE-AS 29 06 732 ist ein Verfahren zur Aktivitätsbestimmung von Peroxidasen mit Hilfe einer Chemilumineszenzreaktion bekannt, welches eine gute Quantenausbeute liefert und es damit ermöglicht, im Rahmen von Enzym-Immuno-Assays die Empfindlichkeit der POD-Bestimmung zu erhöhen und die Dauer der Bestimmung wesentlich herabzusetzen.

Der Chemilumenszenzreaktion liegt die Umsetzung von Luminol mit einer Peroxidverbindung zugrunde; es wird aber anstelle von Luminol das 7-Dimethylaminonaphthalin-1,2-dicarbonsäure-hydrazid eingesetzt, wodurch eine Steigerung der Quantenausbeute der Chemilumineszenz (Chemiluneszenzausbeute) erreicht wird.

Aus Whitehead et al, Nature 305 (1983), 158 - 159, ist es bekannt, die Lumineszenzausbeute der POD-katalysierten Oxidation von Luminol durch einen Zusatz von firefly-Luciferin (Feuerfliegen-Luciferin) um ein Vielfaches zu steigern; eine solche Steigerung ist auch durch einen Zusatz von 6-Hydroxy-benzothiazolen bekannt (vgl. Thorpe et al, Anal. Biochem. 145 (1985) 96 - 100), oder durch einen Zusatz von Phenolderivaten (vgl. Thorpe et al, Clin Chem. 31 (1985) 1335 - 1341; EP-A-0116454).

Mit diesem Verfahren kann zwar eine Steigerung der Quantenausbeute erreicht werden; sie besitzen aber den Nachteil, daß die erforderlichen Zusätze (Aktivatoren) nicht leicht erhältlich sind und/oder aufgrund ihrer schlechten Löslichkeit in Wasser nur in Mischung mit organischen Lösungsmitteln einsetzbar sind.

Aufgabe der vorliegenden Erfindung war deshalb die Bereitstellung eines Verfahrens zur Steigerung der Quantenausbeute bei der Oxidation von Luminol durch Peroxidverbindungen in Gegenwart von POD, das die vorstehend genannten Nachteile vermeidet, und das sich für eine empfindliche und rasche Bestimmung von POD in Enzym-Immun-Assays eignet. Diese Aufgabe wird mit dem erfindungsgemäßen Verfahren gelöst.

Es wurde gefunden, daß sich die Quantenausbeute bei der Oxidation von Luminol durch Peroxidverbindungen in Gegenwart von POD steigern läßt, wenn man die Umsetzung in Gegenwart von Fluorescein durchführt, wobei es einen Konzentrationsbereich von Fluorescein gibt, in dem eine überadditive (synergistische) Quantenausbeute erhalten wird, die größer ist als die Summe der Quantenausbeuten der einzelnen chemilumineszierenden Stoffe.

Gegenstand der Erfindung ist deshalb ein Verfahren gemäß Patentanspruch 1 zur Steigerung der Quantenausbeute bei der Oxidation von Luminol oder 7-Dialkylamino-naphthalin-1,2-dicarbonsäure-hydrazid, worin die Alkylgruppen 1 bis 3 Kohlenstoffatome enthalten, durch Peroxidverbindungen in Gegenwart von POD, das dadurch gekennzeichnet ist, daß man die Umsetzung in Gegenwart von Fluorescein durchführt, wobei die Konzentration des Fluoresceins in einem Konzentrationsbereich liegt, der eine Quantenausbeute ergibt, die größer ist als die Summe der Quantenausbeuten der einzelnen chemilumineszierenden Stoffe.

Zweckmäßige Ausführungsformen dieses Verfahrens sind Gegenstand der Ansprüche 2 bis 6.

Es ist bekannt, daß auch Fluorescein unter der Einwirkung von Wasserstoffperoxid Chemilumineszenzerscheinungen zeigt (vgl. Nilsson und Kearns, J. Phys. Chem. 78 (1974) 1681-1683); dies wird zur Bestimmung von mit Fluorescein markierten Verbindungen in Immuno-Assays verwendet (vgl. EP-A-0054952; EP-A-0046563; DE-A-31 32 491). Aus der Arbeit von B.A. Rusin et al, Khim, Vys. Energ. 11 (1) (1977) 93 - 94 (referiert in CA 86 (1977), S. 595, 130 321 s) ist es bekannt, daß die Chemilumineszenz bei der Oxidation von Luminol durch Fluorescein gequencht wird. Es muß deshalb als überraschend angesehen werden, daß es einen Konzentrationsbereich gibt, in dem eine überaddive (synergistische) Steigerung der Quantenausbeute der Chemilumineszenzreaktion eintritt.

Die Alkylgruppen im 7-Dialkylamino-naphthalin-1,2-dicarbonsäure-hydrazid können verschieden oder vorzugsweise gleich sein, sowie verzweigt oder vorzugsweise geradkettig sein, und sind z.B. Methyl, Äthyl, Propyl und/oder Isopropyl.

Bevorzugt wird das 7-Dimethylamino-naphthalin-1,2-dicarbonsäure-hydrazid eingesetzt.

Als Peroxide eignen sich neben Wasserstoffperoxid auch alle mit POD verträglichen Peroxidverbindungen mit vergleichbarem Oxidationspotential, wie z.B. Alkaliperoxide, Anlagerungsverbindungen von Wasserstoffperoxid an Borsäure (Perborate) oder an Harnstoff; vorzugsweise wird, insbesondere für Enzym-Immuno-Assays, Natriumperborat und in erster Linie $H_2O_2$ eingesetzt.

Das erfindungsgemäße Verfahren wir zweckmäßig bei einem pH-Wert von 7,5 bis 9 durchgeführt, und insbesondere bei einem pH-Wert von ca. 8,5. Als Puffer wird Kaliumphosphatpuffer oder Glycin-NaOH-Puffer bevorzugt, obwohl auch andere übliche Puffer, wie z.B. Tris-HCl, Tris-Sulfat und Tris-Acetat geeignet sind. Die bevorzugte Pufferkonzentration beträgt dabei 10 bis 1000 mmol/l. Die Konzentration von Luminol oder 7-Dialkylaminonaphthalin-1,2-dicarbonsäure-hydrazid, oder von Wasserstoffperoxid liegt, sofern diese Konzentrationen nicht mit dieser Reaktion bestimmt werden sollen, in dem für diese Chemilumineszenzreaktion üblichen Konzentrationsbereich; Luminol oder 7-Dialkylamino-naphthalin-1,2-dicarbonsäure-hydrazid werden zweckmäßigerweise in einer Menge im Bereich von 10 $\mu$mol/l bis 100 mmol/l eingesetzt. Das erfindungsgemäße Verfahren wird zweckmäßigerweise bei den für enzymatische Bestimmungen üblichen Temperaturen, die in der Regel zwischen 20 und 37 °C liegen, durchgeführt, wobei aber für das Verfahren selber auch niedrigere oder höhere Temperaturen in Frage kommen; besonders bevorzugt ist der Temperaturbereich zwischen 22 und 30 °C.

Der Konzentrationsbereich von Fluorescein, in dem eine überaddive (synergistische) Steigerung der Quantenausbeute der Chemilumineszenzreaktion eintritt, kann von der Art und Konzentration der übrigen Reaktionsteilnehmer und den Reaktionsbedingungen abhängen; für die angewandten jeweiligen Bedingungen läßt sich der optimale Bereich aber leicht durch wenige orientierende Versuche bestimmen. Vorzugsweise wird, insbesondere als Indikatorreaktion für Enzym-Immuno-Assays, Luminol und Wasserstoffperoxid in Gegenwart von POD oxidiert; diese Umsetzung wird vorzugsweise in Gegenwart von Fluorescein durchgeführt, wobei mit einer Fluorescein-Konzentration im Bereich von 10 bis 1000 $\mu$mol/l, vorzugsweise von 20 bis 100 $\mu$mol/l gearbeitet wird; in diesem Bereich wird eine Steigerung der Quantenausbeute erhalten, die etwa dem zehnfachen der Ausbeute entspricht, die sich aus der Summe der Quantenausbeuten der jeweils einzelnen chemilumineszierenden Stoffe ergibt.

Aufgrund der hohen Quantenausbeute kann das erfindungsgemäße Verfahren als Indikatorreaktion (Chemilumens-zenz-Test) zur Bestimmung der POD-Aktivität in Immuno-Assays dienen, wobei gegenüber einem Test mit Luminol als alleiniges Substrat eine Steigerung der Empfindlichkeit um das zehnfache erreicht wird. Außer zur Bestimmung von POD ist das erfindungsgemäße Verfahren aber auch zur Bestimmung der an der Reaktion teilnehmenden Peroxide, wie z.B. von Wasserstoffperoxid, oder zur Bestimmung von Luminol oder von 7-Dialkylamino-naphthalin-1,2-dicarbonsäure-hydrazid geeignet.

Mit dem erfindungsgemäßen Verfahren ist es somit z.B. möglich, die Meßzeit für die reine Enzymaktivitätsbestimmung bei den ELISA-Tests bei einer hohen Empfindlichkeit auf ca. 2 bis 3 Minuten herabzusetzen. Die Messung erfolgt vorzugsweise derart, daß die in einem bestimmten Zeitintervall emittierte Lichtmenge gemessen wird.

Gegenstand der Erfindung ist deshalb auch die Verwendung des erfindungsgemäßen Verfahrens zur Bestimmung von POD oder von Luminol oder 7-Dialkylamino-naphthalin-1,2-dicarbonsäure-hydrazid, die insbesondere als Markersubstanzen in Immuno-Assays dienen.

Die POD-Bestimmung unter Verwendung des erfindungsgemäßen Verfahrens kann z.B. im Rahmen einer immunologischen Hapten-Bestimmung durchgeführt werden, bei der man eine bekannte Menge eines mit POD markierten Haptens der zu untersuchenden, eine unbekannte Menge des Haptens enthaltenden Probe zusetzt, die Probe dann mit einem an einen festen Träger gebundenen spezifischen Antikörper des Haptens kontaktiert, die feste von der flüssigen Phase trennt und in einer der beiden Phasen die POD-Aktivität mißt (ELISA-Test). Mit diesen Tests kann z.B. Digoxin, Thyroxin ($T_4$) oder Insulin im Blutserum bestimmt werden, wobei man z.B. nach einer der in der DE-AS 29 06 732 beschriebenen Methoden arbeiten kann.

Ein weiterer Gegenstand der Erfindung ist auch ein Reagenz zur Bestimmung der POD nach dem erfindungsgemäßen Verfahren, das dadurch gekennzeichnet ist, daß es Luminol oder 7-Dialkylamino-naphthalin-1,2-dicarbonsäure-hydrazid, mit jeweils 1 bis 3 Kohlenstoffatomen pro Alkylgruppe, Fluorescein, einen Wasserstoffperoxid-Lieferanten, eine Puffersubstanz (pH 7,5 - 9) und gegebenenfalls ein Sequestrierungsmittel enthält.

Ein bevorzugtes Reagenz für einen Liter Testlösung enthält:

| 10 bis 1000 $\mu$mol | 7-Dialkylamino-naphthalin-1,2-dicarbonsäure-hydrazid oder vorzugsweise Luminol, |
| 10 bis 1000 $\mu$mol | Fluorescein, |
| 50 bis 500 mmol | Kaliumphosphat- oder Glycin-Puffer (pH 7,5 - 9), |
| 10 bis 200 $\mu$mol | $H_2O_2$, und gegebenenfalls |
| 0,01 bis 1 mmol | eines Sequestrierungsmittels. |

Als gegebenenfalls zu verwendendes Sequestrierungsmittel kommen die hierfür bekannten Substanzen, wie Äthylendiamintetraessigsäure (EDTA) und ähnliche, dafür übliche Verbindungen in Betracht. Außerdem kann das Reagenz übliche Stabilisierungsmittel, wie Serumalbumin, Kohlehydrate und dergleichen, enthalten. Als $H_2O_2$-Lieferant können $H_2O_2$ selbst sowie die bekannten, $H_2O_2$ freisetzenden Substanzen, wie z.B. Harnstoff-perhydrat ("festes $H_2O_2$") und ähnliche.

Außer den obengenannten Bestandteilen kann das erfindungsgemäße Reagenz auch mit POD markiertes Hapten sowie einen trägergebundenen spezifischen Antikörper gegen das jeweilige Hapten enthalten, wenn das Reagenz im Rahmen eines Enzym-Immun-Tests verwendet werden soll. Daneben können die anderen, z.B. in derartigen ELISA-Reagenzien üblichen Bestandteile, wie weitere Puffersubstanzen, Stabilisierungsmittel und dergleichen, enthalten sein.

Die nachfolgenden Beispiele erläutern die Erfindung näher, ohne sie darauf zu beschränken. Wenn nicht anders angegeben, beziehen sich Temperaturangaben auf die Celsius-Skala und Prozent- und Mengenangaben auf Gewichtsprozent und Gewichtsteile.

**Beispiele**

Beispiel 1

Dieses Beispiel veranschaulicht den Einfluß der Fluorescein-Konzentration auf die Quantenausbeute.

Alle Versuche wurden mit einem Biolumineszenz-Meßgerät der Fa. Berthold, Wildbad, vom Typ Biolumat LB 9500 durchgeführt. Das Testvolumen betrug 500 $\mu$l, die Temperatur 30 °C.

Es wurden folgende Konzentrationen (Endkonzentrationen im Test) verwendet:

| Wasserstoffperoxid | 0,1 mmol/l |
| Luminol | 25 $\mu$umol/l |
| Peroxidase | 20 mg/l |
| Tris-HCl-Puffer (pH = 8,5) | 90 mmol/l |

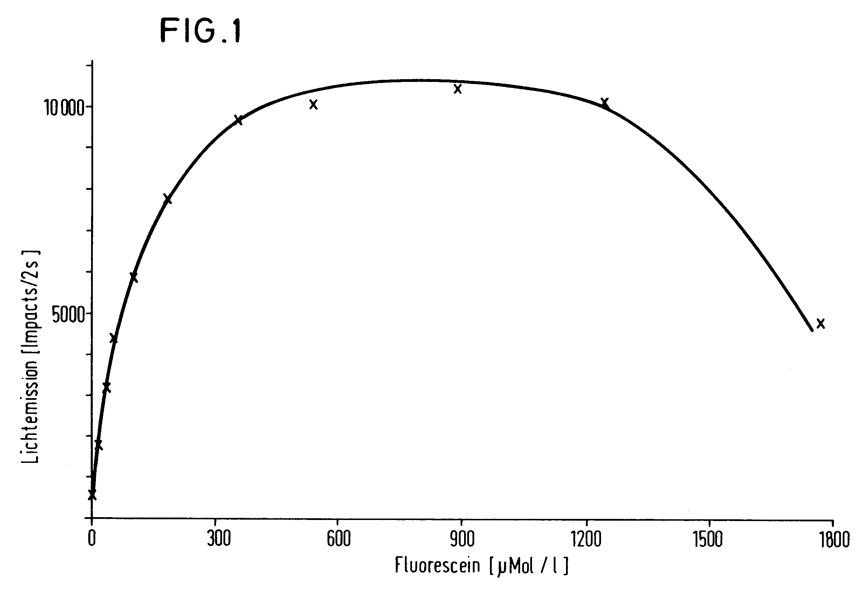

In der Fig. 1 wird die Abhängigkeit der Lichtemission von der Fluorescein-Konzentration graphisch dargestellt. Wie die Fig. 1 zeigt, wird die Lichtemission bei einer Fluorescein-Konzentration zwischen 10 und 1000 $\mu$mol/l um ein Vielfaches aktiviert.

Beispiel 2

Dieses Beispiel veranschaulicht den überadditiven (synergistischen) Effekt beim Zusammenwirken von Luminol und Fluorescein im Vergleich zu einer alleinigen Verwendung von Luminol oder von Fluorescein.

Es wurde mit folgenden Konzentrationen (Endkonzentrationen im Test) gearbeitet:

| Luminol | 0,1 mmol/l |
|---|---|
| Fluorescein | 25 $\mu$mol/l |
| POD | 20 ng/l |
| Wasserstoffperoxid | 0,1 mmol/l |
| Tris-HCl-Puffer (pH = 8,5) | 90 mmol/l |

In der nachfolgenden Tabelle 1 sind die erhaltenen Ergebnisse zusammengestellt. $I_{max}$ bedeutet die Zahl der Lichtemissionen/2 Sekunden.

Tabelle 1

| Nr. | POD | Luminol | $H_2O_2$ | Fluorescein | $I_{max}$(Imp./2sec) |
|---|---|---|---|---|---|
| 1 | + | + | + | + | $3.9 \cdot 10^5$ |
| 2 | + | - | + | + | $5.8 \cdot 10^3$ |
| 3 | + | + | - | + | $8.4 \cdot 10^4$ |
| 4 | + | + | + | - | $2.7 \cdot 10^4$ |
| 5 | + | - | - | + | $7.2 \cdot 10^3$ |
| 6 | + | + | - | - | 432 |
| 7 | - | + | + | + | 25 |

Aus der Tabelle 1 ist der synergistische Effekt beim Zusammenwirken von Luminol und Fluroescein klar erkennbar: Bei Anwesenheit von Luminol und Fluorescein ist die maximale Lichtintensität ($I_{max}$) ca. zehnmal so groß wie die Summe der Intensitäten der Reaktionen unter Anwesenheit von Luminol bzw. von Fluorescein allein (Test Nr. 4 und 2). Der Tabelle ist weiterhin zu entnehmen, daß Fluorescein in Gegenwart von POD auch bereits mit Luftsauerstoff unter Chemilumineszenz reagiert (vgl. Test Nr. 5).

Beispiel 3

Dieses Beispiel zeigt den Einfluß der POD-Konzentration auf die Umsetzung.
Die Messung erfolgte wie in den vorhergehenden Beispielen. Es wurde mit den folgenden Konzentrationen (Endkonzentrationen im Test) gearbeitet:
Luminol          0,1 mmol/l
Wasserstoffperoxid          0,1 mmol/l
Fluorescein          25 $\mu$mol/l
Tris-HCl-Puffer (pH = 8,5)          90 mmol/l
Es wurden die folgenden, in Tabelle 2 zusammengestellten Ergebnisse erhalten.

Tabelle 2

| $C_{POD}$ (MOL/l $\cdot 10^{-10}$) | $I_{max}$ (Imp./2 sec.) | |
|---|---|---|
| | mit Fluorescein | ohne Fluorescein |
| 0 | 40 | 10 |
| 0,25 | 79 | 170 |
| 1,3 | 1580 | 1220 |
| 2,5 | 32500 | 670 |
| 3,1 | 45200 | 540 |
| 6,3 | 433600 | 3580 |
| 8,3 | 514900 | 4790 |
| 13 | größer $10^6$ | 76000 |
| 17 | " | 93200 |
| 25 | " | 339200 |
| 130 | " | größer $10^6$ |

Beispiel 4

Dieses Beispiel zeigt den Einfluß des pH-Werts auf die Reaktion. Es wurde mit den in Beispiel 3 angegebenen Konzentrationen gearbeitet. Die POD-Konzentration betrug 9 x $10^{-10}$ Mol/l, die Konzentration an Tris-HCl-Puffer mit verschiedenen pH-Werten betrug 90 mmol/l. Die pH-Werte von 12,6 wurden durch Zugabe von 1 mol/l Natronlauge erreicht.

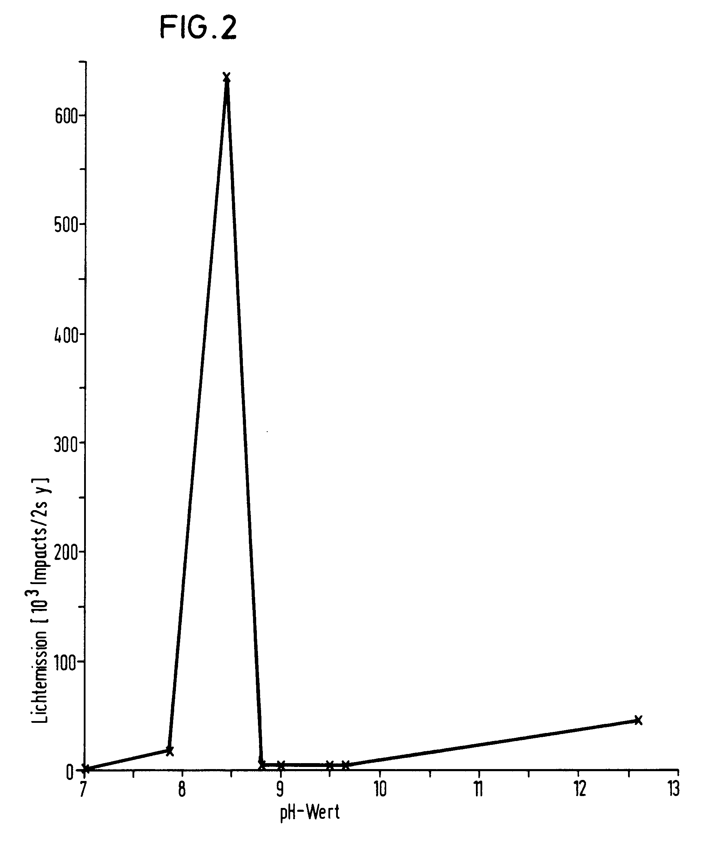

In der Fig. 2 ist der Einfluß des pH-Werts auf die Lichtemission ($I_{max}$) graphisch dargestellt.

Beispiel 5

Es wurde wie in Beispiel 2 gearbeitet, aber anstelle von Wasserstoffperoxid wurde Natriumperborat als Oxidationsmittel verwendet.

In der nachfolgenden Tabelle 3 sind die dabei erhaltenen Ergebnisse zusammengestellt.

Tabelle 3

| Nr. | POD | Perborat | Luminol | Fluorescein | $I_{max}$(Imp./2sec) |
|---|---|---|---|---|---|
| 1 | + | + | + | + | $1.5 \cdot 10^5$ |
| 2 | + | + | - | + | 400 |
| 3 | + | + | + | - | $1.8 \cdot 10^4$ |
| 4 | + | - | - | + | $3.3 \cdot 10^3$ |

Auch aus den Ergebnissen der Tabelle 3 ist der beim Arbeiten in Gegenwart von Luminol und Fluorescein erhaltene überadditive Effekt klar erkennbar.

Beispiel 6

Dieses Beispiel zeigt die Anwendung des erfindungsgemäßen Verfahrens in einem Enzym-Immun-Assay nach dem ELISA-Prinzip zur Bestimmung von Speichel-$\alpha$-Amylase.

Der Test wurde auf folgende Weise durchgeführt:

1. Lumineszenzröhrchen (Lumacuvette P Polystyrene, Recorder Nr. 4960 von Lumac Systems AG, Basel, Schweiz) werden mit einem die humane Speichelamylase spezifisch bindenden monoklonalen Antikörper (5 $\mu$g/ml) gegen humane Speichel-$\alpha$-Amylase in 50 mM Carbonatpuffer (pH = 9,3) (500 $\mu$l) 18 Stunden bei 4 °C inkubiert. Dieser monoklonale Antikörper ist unter der Bezeichnung NCACC 84111305 bei der National Collection of Animal Cell Culture, Porton Down, GB hinterlegt und wird gemäß EP 0150309 hergestellt.

2. Die Röhrchen werden einmal mit 150 mM Phosphatpuffer (pH = 7,2, 145 mM NaCl) und 1 % Rinderserumalbumin (BSA) gewaschen.

3. Es folgt eine Nachbehandlung der Röhrchen (Nachbeschichtung) mit 150 mmol/l Phosphatpuffer (pH 7,2), 145 mmol/l NaCl und 2 % Rinderserumalbumin. Die Röhrchen werden dann eine Stunde bei Raumtemperatur belassen.

4. Die Röhrchen werden wie unter 2. angegeben gewaschen.

5. Humane Speichel-$\alpha$-Amylase-POD-Konjugat wird in verschiedenen Konzentrationen 4 Stunden lang bei 37 °C in den Lumineszenzröhrchen inkubiert (500 $\mu$l).

6. Es wird fünfmal wie unter Punkt 2. beschrieben gewaschen.

7.

a) Zur Entwicklung wird 1 ml ABTS-Reagenz (aus Enzymun-Test Digoxin, Boehringer Mannheim GmbH, Katalog Bestellnummer 199656) in jedes Röhrchen gegeben. Nach genau 9 Minuten wird die Extinktion bei 405 nm gegen nicht inkubiertes ABTS-Reagenz bestimmt.

b) Es werden 500 $\mu$l Lumineszenzreagenz ohne Fluorescein (Endkonzentration siehe Beispiel 2) in jedes Röhrchen gegeben. Nach genau 30 Sekunden oder 17 Minuten wird die Lumineszenz gemessen (Gerät: Biolumineszenz-Meßgerät der Fa. Berthold, Wildbad, des Typs Biolumat LB 9500 T; Integrationszeit 60 sek).

c) Es werden 500 $\mu$l Lumineszenzlösung mit Fluorescein (Endkonzentration siehe Beispiel 2) zugegeben und dann die Lumineszenz wie unter 7. b) beschrieben gemessen.

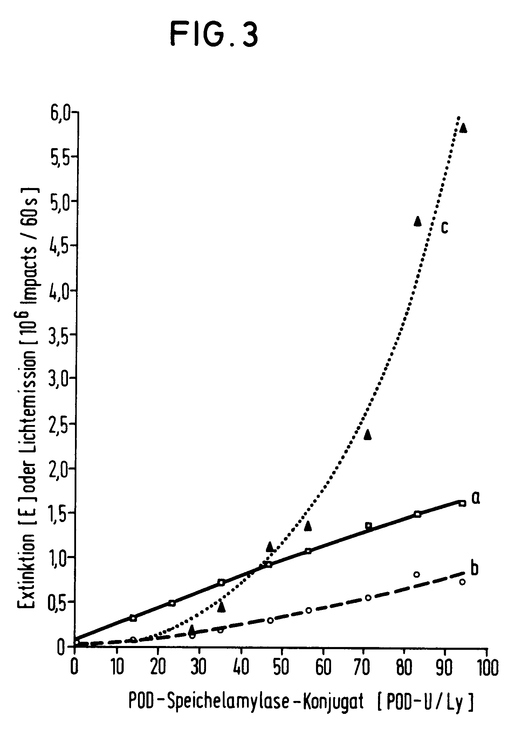

Die Fig. 3 zeigt die mit Indikatorreaktion a), b) und c) erhaltenen Eichkurven. Hierbei ist das erhaltene Meßsignal (im Falle a: Lichtabsorption; in den Fällen b und c: Lichtemission) als Funktion der Konzentration des gebundenen Speichelamylase-POD-Konjugats dargestellt.

Beispiel 7

Synergistischer Effekt bei Verwendung von 7-Dimethylamino-naphthalin-1,2-dicarbonsäurehydrazid (DNH).

Es wurde analog Beispiel 1 gearbeitet. Die Endkonzentrationen im Test betragen:

DNH 0,1 mmol/l
Fluorescein 25 $\mu$mol/l
POD 20 ng/l
Wasserstoffperoxid 0,1 mmol/l
Tris-HCl-Puffer (pH 8,5) 90,0 mmol/l

Tabelle 4

| Nr. | POD | DNH | $H_2O_2$ | Fluorescein | $I_{max}$ (Imp./2 sec) |
|-----|-----|-----|----------|-------------|------------------------|
| 1 | + | + | + | - | $1.1 \cdot 10^4$ |
| 2 | + | + | + | + | $6 \cdot 10^4$ |
| 3 | + | - | + | + | $4.0 \cdot 10^2$ |

Aus Tabelle 4 ergibt sich, daß bei Verwendung von 7-Dimethylamino-naphthalin-1,2-dicarbonsäurehydrazid und Fluorescein ein synergistischer Effekt festgestellt werden kann.

**Patentansprüche**

1. Verfahren zur Steigerung der Quantenausbeute bei der Oxidation von Luminol oder 7-Dialkylaminonaphthalin-1,2-dicarbonsäure-hydrazid, worin die Alkylgruppe 1 bis 3 Kohlenstoffatome enthält, durch Peroxidverbindungen in Gegenwart von Peroxidase (POD), **dadurch gekennzeichnet**, daß man die Umsetzung in Gegenwart von Fluorescein durchführt, wobei die Konzentration des Fluoresceins in einem Konzentrationsbereich liegt, der eine Quantenausbeute ergibt, die größer ist als die Summe der Quantenausbeuten der einzelnen chemilumineszierenden Stoffe.

2. Verfahren nach Anspruch 1, **dadurch gekennzeichnet**, daß man als Peroxidverbindung Wasserstoffperoxid einsetzt.

**3.** Verfahren nach einem der Ansprüche 1 oder 2, **dadurch gekennzeichnet**, daß man die Umsetzung bei einem pH-Wert von 7,5 bis 9 durchführt.

**4.** Verfahren nach Anspruch 3, **dadurch gekennzeichnet**, daß der pH-Wert 8,5 ist.

**5.** Verfahren nach einem der vorhergehenden Ansprüche, **dadurch gekennzeichnet**, daß man Luminol mit Wasserstoffperoxid in Gegenwart von POD oxidiert.

**6.** Verfahren nach einem der vorhergehenden Ansprüche, **dadurch gekennzeichnet**, daß man die Umsetzung in Gegenwart von 10 bis 1000 $\mu$mol/l Fluorescein durchführt.

**7.** Verwendung des Verfahrens nach einem der Ansprüche 1 bis 6 zur Bestimmung von POD, von Wasserstoffperoxid oder von Luminol oder 7-Dialkylaminonaphthalin-1,2-dicarbonsäure-hydrazid.

**8.** Verwendung nach Anspruch 7, **dadurch gekennzeichnet**, daß man POD, Luminol oder 7-Dialkylamino-naphthalin-1,2-dicarbonsäure-hydrazid als Markierungssubstanz in Immuno-Assays bestimmt.

**9.** Reagenz zur Bestimmung von POD, **dadurch gekennzeichnet**, daß es Luminol oder 7-Dialkylamino-naphthalin-1,2-dicarbonsäure-hydrazid, mit jeweils 1 bis 3 Kohlenstoffatomen pro Alkylgruppe, Fluorescein, einen Wasserstoffperoxid-Lieferanten, eine Puffersubstanz (pH 7,5 bis 9) und gegebenenfalls ein Sequestrierungsmittel enthält.

**10.** Reagenz nach Anspruch 9, **dadurch gekennzeichnet**, daß es

| | |
|---|---|
| 10 bis 1000 $\mu$mol/l | Luminol oder 7-Dialkylamino-naphthalin-1,2-dicarbonsäure-hydrazid, |
| 10 bis 1000 $\mu$mol/l | Fluorescein, |
| 50 bis 500 mmol/l | Kaliumphosphat- oder Glycin-Puffer, |
| 10 bis 200 $\mu$mol/l | Wasserstoffperoxid, und gegebenenfalls |
| 0,01 bis 1 mmol/l | eines Sequestrierungsmittels enthält. |

**Claims**

**1.** Process for the increasing of the quantum yield in the case of the oxidation of luminol or 7-dialkylaminonaphthalene-1,2-dicarboxylic acid hydrazide, wherein the alkyl group contains 1 to 3 carbon atoms, by peroxide compounds in the presence of peroxidase (POD) characterised in that one carries out the reaction in the presence of fluorescein, whereby the concentration of the fluorescein lies in a concentration range which gives a quantum yield which is greater than the sum of the quantum yields of the individual chemiluminescing materials.

**2.** Process according to claim 1, characterised in that one uses hydrogen peroxide as the peroxide compound.

**3.** Process according to one of claims 1 or 2, characterised in that one carries out the reaction at a pH value of 7.5 to 9.

**4.** Process according to claim 3, characterised in that the pH value is 8.5.

**5.** Process according to one of the preceding claims, characterised in that one oxidises luminol with hydrogen peroxide in the presence of POD.

**6.** Process according to one of the preceding claims, characterised in that one carries out the reaction in the presence of 10 to 1000 mol/l of fluorescein.

**7.** Use of the process according to one of claims 1 to 6 for the determination of POD, of hydrogen peroxide or of luminol or 7-dialkylaminonaphthalene-1,2-dicarboxylic acid hydrazide.

**8.** Use according to claim 7, characterised in that one determines POD, luminol or 7-dialkylaminonaphthalene-1,2-dicarboxylic acid hydrazide as labelling substance in immuno-assays.

9. Reagent for the determination of POD, characterised in that it contains luminol or 7-dialkylaminonaphthalene-1,2-dicarboxylic acid hydrazide, with, in each case, 1 to 3 carbon atoms per alkyl group, fluorescein, a hydrogen peroxide provider, a buffer substance (pH 7.5 to 9) and possibly a sequestering agent.

10. Reagent according to claim 9, characterised in that it contains:

| | |
|---|---|
| 10 to 1000 $\mu$mol/l | luminol or 7-dialkylaminonaphthalene-1,2-dicarboxylic acid hydrazide, |
| 10 to 1000 $\mu$mol/l | fluorescein, |
| 50 to 500 mmol/l | potassium phosphate or glycine buffer, |
| 10 to 200 $\mu$mol/l | hydrogen peroxide, and possibly |
| 0.01 to 1 mmol/l | of a sequestering agent. |

**Revendications**

1. Procédé pour augmenter le rendement quantique lors de l'oxydation du luminol ou d'un hydrazide d'acide 7-dialkylaminonaphtalène-1,2-dicarboxylique, le groupe alkyle contenant 1 à 3 atomes de carbone, par des composés peroxydiques en présence de peroxydase (POD), caractérisé en ce que l'on effectue la réaction en présence de fluorescéine, la concentration de la fluorescéine étant située dans un domaine de concentration qui donne un rendement quantique supérieur à la somme des rendements quantiques des différentes substances chimiluminescentes.

2. Procédé selon la revendication 1, caractérisé en ce que l'on utilise l'eau oxygénée comme composé peroxydique.

3. Procédé selon l'une des revendications 1 ou 2, caractérisé en ce que l'on effectue la réaction à un pH de 7,5 à 9.

4. Procédé selon la revendication 3, caractérisé en ce que le pH est égal à 8,5.

5. Procédé selon l'une des revendications précédentes, caractérisé en ce que l'on oxyde le luminol avec l'eau oxygénée en présence de POD.

6. Procédé selon l'une des revendications précédentes, caractérisé en ce que l'on effectue la réaction en présence de 10 à 1000 $\mu$mol/l de fluorescéine.

7. Utilisation du procédé selon l'une des revendications 1 à 6 pour la détermination de la POD, de l'eau oxygénée ou du luminol ou de l'hydrazide d'acide 7-dialkylaminonaphtalène-1,2-dicarboxylique.

8. Utilisation selon la revendication 7, caractérisée en ce que l'on détermine la POD, le luminol ou l'hydrazide d'acide 7-dialkylaminonaphtalène-1,2-dicarboxylique en tant que substance de marquage dans des immunodosages.

9. Réactif pour la détermination de la POD, caractérisé en ce qu'il contient du luminol ou un hydrazide d'acide 7-dialkylaminonaphtalène-1,2-dicarboxylique, avec à chaque fois 1 à 3 atomes de carbone par groupe alkyle, la fluorescéine, un générateur d'eau oxygénée, une substance tampon (pH 7,5 à 9) et éventuellement un agent séquestrant.

10. Réactif selon la revendication 9, caractérisé en ce qu'il contient

| | |
|---|---|
| 10 à 1000 $\mu$mol/l | de luminol ou d'hydrazide d'acide 7-dialkylaminonaphtalène-1,2-dicarboxylique, |
| 10 à 1000 $\mu$mol/l | de fluorescéine, |
| 50 à 500 mmol/l | de tampon phosphate de potassium ou glycine, |
| 10 à 200 $\mu$mol/l | d'eau oxygénée, et éventuellement |
| 0,01 à 1 mmol/l | d'un agent séquestrant. |

FIG.1

# FIG.2

# FIG.3